# EUROPÄISCHE PATENTSCHRIFT

(11) **EP 2 207 582 B1**
(45) Veröffentlichungstag und Bekanntmachung des Hinweises auf die Patenterteilung: **12.06.2013**
(21) Anmeldenummer: 08804946.5
(22) Anmeldetag: 01.10.2008
(51) Int. Cl.: A61M 5/172, A61B 5/00

(54) **SYSTEM UND VERFAHREN ZUR ÜBERWACHUNG UND REGELUNG VON BLUTGLUKOSEWERTEN**
SYSTEM AND METHOD FOR MONITORING AND REGULATING BLOOD GLUCOSE LEVELS
SYSTEME ET PROCEDE POUR CONTROLER ET REGULER DES TAUX DE GLYCEMIE

(30) Priorität: 02.10.2007 DE 102007047351
(43) Veröffentlichungstag der Anmeldung: 21.07.2010
(73) Patentinhaber: B. Braun Melsungen AG, 34212 Melsungen (DE)
(72) Erfinder: LAUER, Hans-Martin, 80997 München (DE); WUFKA, Matthias, 80636 München (DE); HÖRNIG, Sebastian, 85258 Weichs (DE); RÖTHLEIN, Doris, 34121 Kassel (DE)
(74) Vertreter: Winter, Brandl, Fürniss, Hübner, Röss, Kaiser, Polte - Partnerschaft
(86) Internationale Anmeldenummer: PCT/EP2008/063122
(87) Internationale Veröffentlichungsnummer: WO 2009/047178

(56) Entgegenhaltungen:
- DE-A1- 19 756 872
- US-A- 4 526 568
- US-A1- 2002 040 208
- US-A1- 2005 277 890
- US-A1- 2007 112 298

## Beschreibung

Die Erfindung betrifft ein System und ein Verfahren zur Überwachung und Regelung von Blutglukosewerten in einem Blutkreislauf gemäß den Oberbegriffen der Patentansprüche 1 und 9.

Aus EP 0 461 207 B1 ist beispielsweise ein System zur Überwachung des Glukosespiegels im Körpergewebe eines Patienten bekannt, wobei das System darauf ausgerichtet ist, mittels implantierbarer glukoseempfindlicher lebender Zellen, die im Stande sind, ein elektrisches oder optisches Signal zu produzieren in Antwort auf die Glukosekonzentration in dem Medium, die elektrischen und optischen Signale mittels außerhalb der lebenden Zellen gelegenen Mittel zu detektieren. Derartige Zellen sind für die Verwendung bei Patienten, die auf Intensivstation liegen, aufgrund der erforderlichen Implantation weniger geeignet.

Aus EP 1 185 321 B1 ist ein Infusionssystem mit geschlossenem Regelkreis für die Infusion eines Fluids in einem Verbraucher bekannt, wobei ein Sensorsystem die Glukosekonzentration überwacht und das von ihm abgegebene Sensorsignal eine Reglereingabe erzeugt, die von einem Regler zur Erzeugung von Befehlen benutzt wird, welche an ein Liefersystem, beispielsweise für Insulin, weitergeleitet werden. Bei dem Regler handelt es sich um einen spezifischen Proportional-Integral-Differiential-Regler. Über den eigentlichen Glukosewert hinausgehende Werte, die den Glukosewert beeinflussen können, werden bei diesem System gar nicht oder nur wenig berücksichtigt.

US2007/0112298A1 offenbart eine externe Infusionspumpe, die über gemessene Glukosewerte gesteuert wird und eine Ausgabeeinrichtung umfasst, auf der behandlungsrelevante Daten ausgegeben werden.

In EP 1 458 435 B1 wird ein System zur Ausgabe von Medikamenten mit einer Infusionspumpe und einer Steuerung mit einem Algorithmus zur Steuerung der Medikamentenabgabe der Infusionspumpe beschrieben. Die Steuerung weist eine Vielzahl von Medikamentenabgabeprofilen zur Abgabe eines Medikaments aus dem Vorratsbehälter auf. Die Steuerung zeigt eine Vielzahl an Aussetzfunktionen, bei denen sich mindestens eines aus der Vielzahl von Medikamentenabgabeprofilen separat zeitweilig außer Kraft setzen lässt. Ein erstes Abgabeprofil, mit dem ein erstes Medikament abgegeben wird, kann ausgesetzt werden, während ein zweites Abgabeprofil, mit dem ein zweites Medikament abgegeben wird, fortfährt. Insofern wird hier der Schwerpunkt auf eine Kombination einer Mehrzahl von Abgabeprofilen gerichtet, wobei die als den Glukosespiegel beeinflussende Größe Insulin betrachtet wird. Ein für Patienten auf Intensivstation, die eine künstliche Ernährung benötigen, geeignete Steuerung von Medikamenten wird hier nicht beschrieben.

Demzufolge liegt der vorliegenden Erfindung die Aufgabe zugrunde, ein System und ein Verfahren zur Überwachung und Regelung von Blutglukosewerten in einem Blutkreislauf zur Verfügung zu stellen, bei dem eine Überwachung und Regelung auch bei Blutkreisläufen von Patienten, die auf Intensivstation liegen und somit künstliche Ernährung benötigen, in Abhängigkeit von den Parametern der künstlichen Nahrung, welche den Glukosespiegel beeinflussen, zu ermöglichen.

Diese Aufgabe wird systemseitig durch die Merkmale des Patentanspruches 1 und verfahrensseitig durch die Merkmale des Patentanspruches 9 gelöst.

Ein wesentlicher Punkt der Erfindung liegt darin, dass bei einem System zur Überwachung und Regelung von Blutglukosewerten in einem Blutkreislauf folgende Komponenten vorhanden sind:
- Eine Eingangseinrichtung zum Empfangen von mindestens einem gemessenen im Blutkreislauf bestehenden Blutglukosewert, von mindestens einem mit mindestens einer Insulinzuführeinrichtung dem Blutkreislauf bisher zugeführten Insulinwert und/oder von mindestens einem Nahrungswert einer mit mindestens einer Nahrungszuführeinrichtung dem Blutkreislauf bisher zugeführten gegebenenfalls künstlichen Nahrung,
- einer Berechnungseinrichtung zum Berechnen eines neuen Insulinwertes bzw. einer neuen Insulinrate und gegebenenfalls eines neuen Nahrungswertes in Abhängigkeit ihres Einflusses auf Blutglukosewerte und in Abhängigkeit von dem zuvor gemessenen Blutglukosewert und
- eine Ausgabeeinrichtung zum Ausgeben der neuen Insulinwerte oder der neuen Insulinrate und gegebenenfalls Nahrungswerte, die enteral oder parenteral zugeführt werden können.

Ein erfindungsgemäßes Verfahren zur Überwachung und Regelung von Blutglukosewerten in dem Blutkreislauf weist folgende Schritte auf:
- Starten mindestens einer Nahrungszuführeinrichtung zum direkten oder indirekten Zuführen vorzugsweise künstlicher Nahrung in den Blutkreislauf,
- Ermitteln mindestens eines Nahrungswertes der vorzugsweise künstlichen Nahrung aus einer Mehrzahl an von der Nahrungszuführeinrichtung an eine Steuerungseinrichtung übermittelten Daten,
- Übermittlung von gemessenen in dem Blutkreislauf bestehenden Blutglukosewerten in die Steuerungseinrichtung,
- Berechnen eines Insulinwertes bzw. einer Insulinrate und gegebenenfalls eines Nahrungswertes in Abhängigkeit von ihrem Einfluss auf Blutglukosewerte und in Abhängigkeit von dem zuvor gemessenen Blutglukosewert mittels einer Berechnungseinrichtung und
- Ausgeben der berechneten neuen Insulinwerte bzw. Insulinrate und gegebenenfalls der berechneten neuen Nahrungswerte mittels einer Ausgabeeinrichtung.

In die Berechnungseinrichtung können also als Eingangsdaten, Daten zu Nahrungswerten einer parenteralen oder/und enteralen Ernährung, Blutglukosewerte, Patientendaten, wie Gewichtsangaben und dergleichen, und Insulinwerte eingegeben werden. Als Ausgangsdaten werden Insulinwerte bzw. eine neue Insulinrate, der Zeitpunkt der nächsten Messung, eventuelle Glukoseplausibilitätsüberprüfungswerte und gegebenenfalls Nahrungswerte von der Berechnungseinrichtung ausgegeben.

Insbesondere der Zeitpunkt der nächsten Messung ist als wichtiger Ausgangswert zu betrachten, wobei hierbei vorzugsweise eine Zeitspanne von 0,5-4 h ab der zuletzt gemachten Messung verwendet wird. Sofern eine derartige Messung innerhalb der angegebenen Zeitspanne nicht stattfindet, findet eine Alarmierung des Personals statt.

Ein derartiges System und ein derartiges Verfahren können vorteilhaft bei Patienten auf Intensivstation eingesetzt werden, die auf künstliche Ernährung angewiesen sind, ohne dass die Gefahr einer Über- oder Unterdosierung der Menge an zugeführten Insulin aufgrund der Vernachlässigung der Einflussfaktoren durch die zugeführte künstliche Nahrung entsteht. Der Blutkreislauf einer Person kann schnelle Reaktionen auf falsche Insulinraten hervorrufen und hierdurch bei einer Zufuhr von Insulin ohne oder bei zu geringer gleichzeitiger Zufuhr von Nahrung in einen lebensbedrohlichen Zustand der Hypoglykemie geraten. Hierbei ist zu berücksichtigen, dass die dem Patienten zugeführte Nahrung für den Blutzuckerspiegel relevante Parameter aufweisen, wie insbesondere die Kohlenhydratkonzentration der Nahrung und die kontinuierlich in Kohlenhydrate pro Zeiteinheit (Kohlenhydrat-Rate) oder als Bolus verabreichte Kohlenhydratmenge.

Bei einer Berücksichtigung derartiger Kohlenhydratwerte in der Berechnung eines neuen Insulinwertes findet eine gegenseitig abhängige Betrachtung der Nahrungswerte, der Insulinwerte und der sich daraus ergebenden Blutglukosewerte statt, die eine ungewollte Reaktion des menschlichen Körpers auf zugeführte Stoffe vermeiden kann. Ebenso kann die Menge und die Dauer des zugeführten Insulins in Abhängigkeit von dem Umstand, ob der menschliche Körper Nahrung mit geänderten Nahrungswerten zugeführt bekommen soll, berechnet werden und somit eine Abstimmung auf den Typ der zugeführten künstlichen Nahrung, also die Nahrungsart, bei der Berechnung der Insulinwerte erfolgen. Es kann sowohl eine enterale als auch eine parenterale Ernährung erfolgen.

Ebenso kann bei einem derartigen System bzw. Verfahren berücksichtigt werden, dass eine Aussetzung der künstlichen Ernährung stattfindet, die sich auf den Blutglukosespiegel auswirkt. Eine daraufhin neu berechnete Insulinrate führt dazu, dass die durch die aussetzende künstliche Ernährung negativ auf den Blutglukosewert stattfindende Auswirkung ausgeglichen werden kann, so dass eine Überreaktion des Blutkreislaufes aufgrund der drohenden nachteilhaften Glukosewerte ausbleibt. Bei einem stattfindenden Ernährungsstopp beispielsweise durch Unterbrechung der parenteralen oder enteralen Ernährung und dem weiterhin aufrechterhaltenen Zuführen von Insulin mit der gleichen Insulinrate besteht die Gefahr der Hypoglykemie aufgrund einer Überdosierung mit Insulin.

Die künstliche Ernährung kann sowohl kontinuierlich als auch bolusartig erfolgen und automatisiert in Antwort auf die zuvor berechneten neuen Nahrungswerte und/oder neuen Insulinwerte oder manuell, indem ein Bedienungspersonal die zuvor berechneten Werte von einer Anzeigeeinrichtung abliest, oder selbst erzeugte Werte vorgibt, zugeführt werden. Vorrangig wird jedoch die Ermittlung von Insulinwerten bzw. einer Insulinrate aus den zuvor in der Berechnungseinrichtung eingegangenen Werten zu der Nahrung und der Blutglukose angestrebt.

Selbstverständlich können die Nahrungswerte von zugeführter Nahrung auch unabhängig von berechneten Werten aufgrund einer individuell gewünschten Änderung der Ernährung verändert werden. Insofern ist die künstliche Ernährung als in die Berechnungseinrichtung eingehender Eingangswert zu betrachten, der wiederum einen neuen Insulinwert bzw. eine neue Insulinrate unter Berücksichtigung des Blutglukosewertes als Ausgangswert hervorruft.

Die Eingangs- und die Ausgabeeinrichtung sind in einer Steuereinrichtung mit Sende- und Empfangseinrichtung zusammengefasst. Eine derartige Steuereinrichtung (Space Control) steht in direkter Verbindung mit der Berechnungseinrichtung (Space Com). Alternativ können beide Einrichtungen in einer gemeinsamen Vorrichtung integriert sein.

Die Ausgabeeinrichtung ist an die Anzeigeeinrichtung angeschlossen, die in erster Linie dazu dient, die neu berechneten Insulinwerte und gegebenenfalls Nahrungswerte anzuzeigen und abzulesen und ggf. mittels eines Touchscreens zugleich eine Eingangseinrichtung dazustellen, in der die gemessenen Blutglukosewerte und ggf. gewünschte neue Nahrungswerte und/ oder neue Insulinwerte in das System eingegeben werden können. Ein derartiges User Interface steht in direktem Kontakt mit einer Therapiesteuerungseinheit, die innerhalb der Steuereinrichtung angeordnet ist und für die Steuerung und den Austausch von Daten, die von und zu der Berechnungseinrichtung gesendet werden, u.a. steuernd verantwortlich ist.

Die Ausgabeeinrichtung ist vorzugsweise mit der Nahrungszuführeinrichtung und der Insulinzuführeinrichtung gekoppelt, sofern eine automatisierte Zuführung von Insulin und künstlicher Nahrung mit den neu berechneten Insulinwerten und Nahrungswerten stattfinden soll.

Gemäß einer bevorzugten Ausführungsform wird ein Abfragesignal von der Steuereinrichtung zur Abfrage der Nahrungswerte an die Nahrungszuführeinrichtung zu mindestens einem vorbestimmbaren Zeitpunkt gesendet. Dieses Abfragesignal dient dazu, dass als abgefragte Nahrungswerte beispielsweise die aktuelle Förderrate der als Pumpe ausgestalteten Nahrungszuführeinrichtung, die Nahrungsart, also das Nahrungsmedikament, welches verabreicht wird, und deren Kohlenhydratekonzentration der Steuereinrichtung bekannt werden, so dass diese eine Kohlenhydratrate als relevante Nahrungswerte aus diesen Daten berechnen kann. Anschließend findet eine Übermittlung der Kohlenhydratrate an die Berechnungseinrichtung zum Berechnen der neuen Insulinwerte ggf. neuer Nahrungswerte statt.

Anstelle der bereits beschriebenen Anzeigeeinrichtung in Form eines Touchscreens, die zugleich eine Eingabe der verschiedenen Daten, wie beispielsweise der gemessenen Blutglukosedaten oder weiterer personenspezifischer Werte, durch das Bedienungspersonal zulässt, kann eine separate Eingabevorrichtung zum Eingeben der gemessenen oder berechneten Blutglukosewerte und/ oder Nahrungswerte und weiterer personenspezifischer Werte wie Körpergewicht, Personenalter und dergleichen Daten als Eingangseinrichtung verwendet werden. Beispiele hierfür sind herkömmliche Tastaturen oder bedienbare Cursor-Steuerungseinrichtungen, wie beispielsweise eine Maus.

Die wesentlichen über eine derartige Eingabevorrichtung eingegebenen und für die Berechnung des neuen Insulinwertes bzw. der neuen Insulinrate und gegebenenfalls neuer Nahrungswerte relevanten Werte sind der Blutglukosewert, der mittels einer separaten Einrichtung in dem Blutkreislauf, beispielsweise eines Patienten, oder außerhalb gemessen werden kann, eine Änderung der künstlichen Ernährung, woraus sich eine Veränderung der Nahrungswerte, die gewünscht ist, ergibt und/oder eine Änderung des Patientengewichtes. Die Eingabe dieser Daten/Werte führt in jedem Fall zu einer Berechnung neuer Insulinwerte bzw. Insulinraten und gegebenenfalls neuer Nahrungswerte, um hierdurch eine Einstellung des Blutkreislaufes auf die geänderten Daten und eine neue Einstellung der gegenseitigen Abhängigkeit von Insulinwerten, Blutglukosewerten und Nahrungswerten zu erhalten. Selbstverständlich wird bei einer beispielsweise stattgefundenen und gewünschten Änderung der Nahrungswerte durch eine Bedienungsperson, welche dann über die Eingabeeinrichtung dem System bekannt gemacht wird, nicht ein neuer Nahrungswert, sondern lediglich ein neuer Insulinwert berechnet.

Gemäß einer bevorzugten Ausführungsform ist eine Alarmierungseinrichtung in der Steuereinrichtung zum optischen und/oder akustischen Alarmieren eines Bedieners für die zeitabhängig fällige Messung des Blutglukosewertes angeordnet. Eine derartige Alarmierungseinrichtung kann ebenso bzw. zusätzlich eine Alarmierung bei einem Ausfall der künstlichen Ernährung aufgrund beispielsweise eines technischen Defektes auslösen. Ebenso kann eine unerwünschte Veränderung in der Ernährung alarmiert werden.

Eine für die Alarmierung ausschlaggebende Ernährungsänderung, welche unerwünscht ist, teilt sich in drei verschiedene Fälle auf: Die Ernährung kann unerwünscht aussetzen, wobei es sich sowohl um eine enterale als auch um eine parenterale Ernährung handeln kann. Alternativ kann die Ernährungsrate der enteralen oder parenteralen Nahrung geändert werden. Als dritte Möglichkeit findet eine Ernährungsänderung dann statt, wenn ein Einmalartikel der Nahrungsversorgungseinrichtung(en) gewechselt wird.

Es wird jeweils in Abständen von ca. fünf Minuten, vorzugsweise fünf Minuten nach einer Änderung der Ernährung alarmiert, wenn nicht zwischenzeitlich eine Eingabe zur Berechnung der neuen Insulinrate an der Steuerungseinrichtung bzw. deren Eingabevorrichtung erfolgt ist.

Eine Alarmierung für die fällige Messung des Blutglukosewertes kann beispielsweise gemäß folgendem Ablauf erfolgen: Ein Voralarm findet 10 Minuten vor der eigentlichen nächstfälligen Messung statt. Ein Zeitabstand für zu erfolgende Messungen kann beispielsweise in einem Bereich von 0,5 - 4 Stunden liegen. Ein derartiges Alarmsignal kann auch für zehn Minuten stummgeschaltet werden.

Zum Zeitpunkt der fälligen Messung wird dann der Alarmton wiederholt bzw. wieder angeschaltet. Dies geschieht ebenso nach weiteren 10 Minuten und 20 Minuten. Auch hierfür kann der Alarmton für 10 Minuten stummgeschaltet werden.

Nach 30 Minuten findet ein weiterer Alarm mit der Aufforderung statt, dass der Benutzer die Insulinpumpe stoppen muss, da ein automatischer Modus das System verlassen wird und somit kein automatisierter Stopp der Insulinpumpe stattfindet. Sofern ohne Blutglukosemessung fortgefahren wird, muss in den manuellen Modus des Systems gewechselt werden. Dies beinhaltet, dass in dem manuellen Modus vom Bedienungspersonal eine neue Dosisrate für das zuzuführende Insulin gesetzt wird.

Alternativ kann ein automatischer Abschaltmodus bezüglich eines automatisierten Stopps der Insulinpumpe vorliegen. Es liegt hierbei eine Kommunikationsverbindung zwischen der Insulinpumpe und der Space-Control vor, um so eine durch die Space-Control verursachte Beendigung der Insulinpumpentätigkeit zu ermöglichen. Ein derartiger automatisierter Stopp-Modus kann als System betrieben werden, welches einen Vorschlag für den Stopp der Insulinpumpe ausgibt, wobei das System jederzeit von einem Arzt bzw. einer Krankenschwester, die diesen Vorschlag nicht folgen möchten, manuelle beeinflusst werden kann, um anschließend wieder in einen automatisierten Modus zurückzugehen.

Sofern nicht ohne, sondern mit der Blutglukosemessung fortgefahren wird, wird von dem Bedienungspersonal eine Eingabe eines neuen Glukosewertes und ein Festlegen der vorgeschlagenen Insulinrate an der Insulinpumpe durchgeführt. Nun findet wieder ein automatischer Modus des Systems statt.

Das erfindungsgemäße Verfahren zur Überwachung und Regelung von Blutglukosewerten in einem Blutkreislauf weist die Schritte des Startens mindestens einer Nahrungszuführeinrichtung zum Zuführen künstlicher Nahrung in den Blutkreislauf, des Ermittelns mindestens eines Nahrungswertes der künstlichen Nahrung aus einer Mehrzahl an eine Steuerungseinrichtung übermittelte Daten, des Übermittelns von mindestens einem gemessenen Blutglukosewert in die Steuerungseinrichtung und des Berechnens eines Insulinwertes und ggf. eines Nahrungswertes in Abhängigkeit ihres Einflusses auf Blutglukosewerte und in Abhängigkeit von dem zuvor gemessenen Blutglukosewert mittels einer Berechnungseinrichtung auf. Zudem wird der berechnete neue Insulinwert und ggf. berechnete neue Nahrungswerte mittels einer Ausgabeeinrichtung ausgegeben.

Die berechneten Insulinwerte und die ggf. die berechneten Nahrungswerte können entweder automatisiert an eine Insulinzuführeinrichtung und die Nahrungszuführeinrichtung oder mittels Eingabe durch das Bedienungspersonal an diese Zuführeinrichtungen übertragen und gegebenenfalls bestätigt werden.

Es handelt sich bei beiden Zuführeinrichtungen um Pumpen, wobei die Nahrungszuführeinrichtung vorzugsweise aus einer Pumpe für enterale Nahrungszuführung und aus einer Pumpe parenteraler Nahrungszuführung besteht.

Zur Berechnung der erforderlichen Insulinwerte und ggf. Nahrungswerte wird ein Patientenmodell verwendet, welches verschiedene personenspezifische Daten, wie das Alter, die bisherige Insulinrate, welche bisher verabreicht wurde, das Körpergewicht und weitere Parameter in seinem Berechnungsmodell berücksichtigt.

Weitere vorteilhafte Ausführungsformen ergeben sich aus den Unteransprüchen.

Vorteile und Zweckmäßigkeiten sind der nachfolgenden Beschreibung in Verbindung mit der Zeichnung zu entnehmen. Hierbei zeigen:
- Fig. 1: Ein Funktionsablaufdiagramm in schematischer Darstellung, welches einen Teil des erfindungsgemäßen Verfahrens wiedergibt;
- Fig. 2: ein Funktionsablaufdiagramm eines Abschnitts des erfindungsgemäßen Verfahrens;
- Fig. 3: ein schematisiertes Ablaufdiagramm des erfindungsgemäßen Verfahrens;
- Fig. 4: ein schematisiertes Ablaufdiagramm eines Ausschnitts des erfindungsgemäßen Verfahrens;
- Fig. 5: ein Funktionsablaufdiagramm des erfindungsgemäßen Verfahrens in einer weiteren Darstellung;
- Fig. 6: in einer schematischen Darstellung das erfindungsgemäße System, und
- Fig. 7: in einem Diagramm die zeitlich abhängigen Verläufe der Blutglukose zur Verwendung in dem erfindungsgemäßen System.

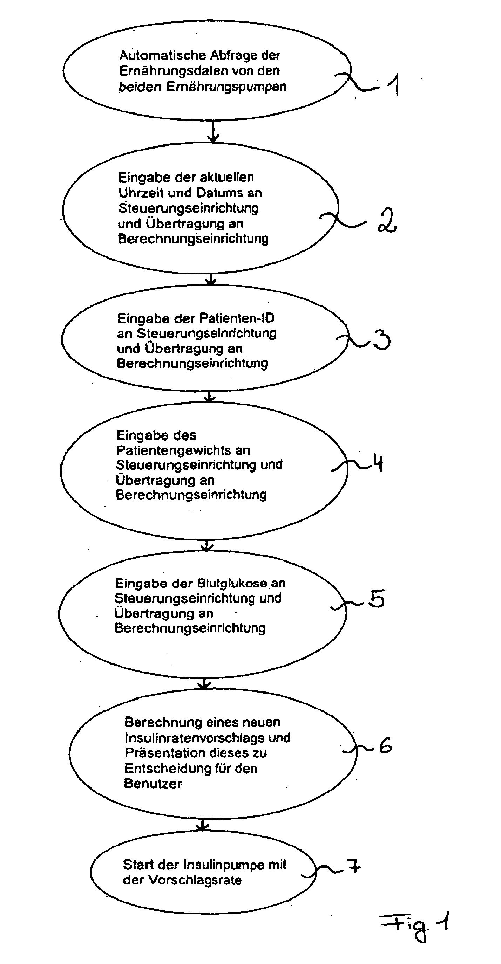

In Fig. 1 wird ein Abschnitt des erfindungsgemäßen Verfahrens in einem Ablaufdiagramm gemäß einer Ausführungsform der Erfindung dargestellt. Hierbei handelt es sich um eine Initialisierungsphase des Verfahrens. In einer derartigen Initialisierungsphase werden alle benötigten Daten automatisiert, abgefragt oder manuell, zumindest teilweise, eingegeben und ein erster Insulinwertvorschlag präsentiert. Danach wartet das erfindungsgemäße System auf weitere Anlässe, die zu einer erneuten Berechnung der Insulinrate oder einer Alarmierung führen können. Beispielsweise sind hier Änderungen in der Ernährung, ein neuer Blutglukosewert, ein Ablauf des Zeitgebers (Timer) für die nächste Blutglukosemessung oder eine manuelle Änderung der Insulinrate zu nennen.

Eine Steuerungseinrichtung (Space Control) 1 sendet ein Abfragesignal automatisiert an zwei hier nicht näher dargestellte Ernährungspumpen, die zur parenteralen und zur enteralen Ernährung dienen. Das Abfragesignal liefert Informationen hinsichtlich des Zustandes der Ernährungspumpen und hinsichtlich therapierelevanter Daten der in den Pumpen vorhandenen Nahrungsart von den Ernährungspumpen an die Steuerungseinrichtung. Insbesondere der Kohlehydratwert und die aktuelle Förderrate sind hierbei als wichtige Daten zu der Nahrungsart abzufragen.

Anschließend oder zeitgleich findet durch eine Bedienperson eine Eingabe weiterer Daten mittels einer Eingangseinrichtung in Form einer Eingabevorrichtung wie einer Tastatur, oder/und einem Bildschirm mit Maus statt. Beispielsweise wird die aktuelle Uhrzeit und das Datum in die Steuereinrichtung zur Übertragung an die Berechnungseinrichtung (Space Com) gemäß Schritt 2 eingegeben. Anschließend findet die Eingabe 3 der Patientenidentifikationsnummer (Patienten- ID) an der Steuerungseinrichtung (Space Control) zur Übertragung an die Berechnungseinrichtung statt.

In einem weiteren Schritt 4 wird das Patientengewicht eingegeben und an die Berechnungseinrichtung übertragen.

In einem Schritt 5 findet die Eingabe und Übertragung der Blutglukosewerte statt.

In einem Schritt 6 wird in der Berechnungseinrichtung eine neue Insulinrate auf Basis der oben angegebenen Werte und des in der Berechnungseinrichtung gespeicherten Patientenmodelles berechnet und diese neue Insulinrate für eine ausstehende Entscheidung dem Bedienungspersonal mittels einer Anzeigeeinrichtung angezeigt. Ein Start der Berechnungseinrichtung nach Eingabe sämtlicher Daten findet automatisiert mittels der Steuereinrichtung statt.

Das Bedienungspersonal kann aus medizinischer Sicht nochmals beurteilen, ob die angezeigte Insulinrate sinnvoll erscheint und anschließend diese Insulinrate an der Insulinpumpe eingeben (Schritt 7).

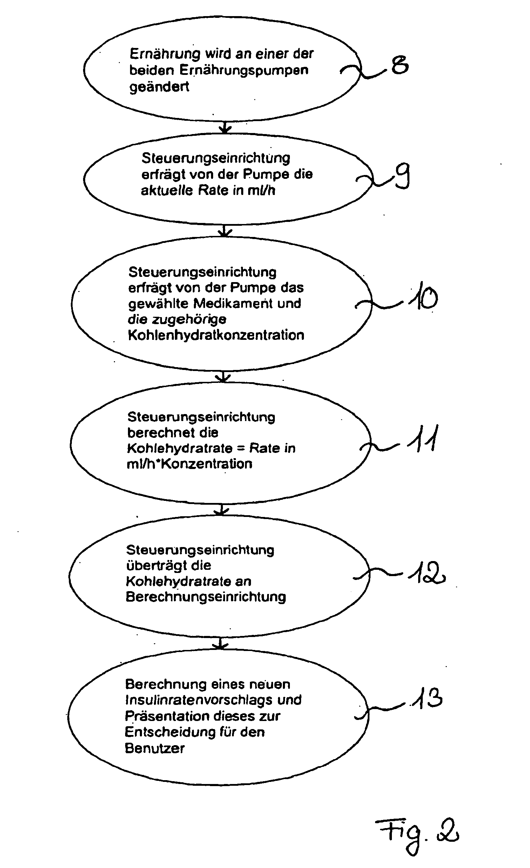

In Fig. 2 ist ein Abschnitt des erfindungsgemäßen Verfahrens als Flußdiagramm wiedergegeben. Hierbei handelt es sich um die automatische Einbindung der Ernährung und der gelieferten Nahrungswerte bei deren Änderung in die Berechnung des neuen Insulinwertes.

Demzufolge wird in Fig. 1 der initiale Start des Verfahrens dargestellt, wohingegen in Fig. 2 die Einbindung der Ernährung, also die Reaktion des Blutkreislaufes auf Änderungen während eines laufenden Verfahrens, dargestellt wird.

Gemäß Schritt 8 wird die Nahrungsart oder deren Förderungsrate oder ein weiterer Nahrungsparameter durch den Bediener an der Nahrungszuführungseinrichtung geändert. In derartigen Nahrungszuführeinrichtungen in Form von Ernährungspumpen kann das Nahrungsmedikament aus der spezifischen Nahrungsmedikamentendatenbank ausgewählt werden.

Der Nahrungsmedikamentendatensatz enthält die Information über Teilkonzentrationen des Ernährungsmedikaments. Die Nahrungsmedikamentendatenbank wird mit einem separatem PC-Programm erstellt und anschließend auf eine Einrichtung in der Ernährungspumpe geladen. Beispielsweise kann ein Datensatz für ein Ernährungsmedikament wie folgt aussehen:

| Name | Nutricomp Standard Neutral |
|---|---|
| Energie | 421 kJ/100ml |
| Kohlenhydrate | 13,8 g/100 ml |
| Eiweiß | 3,8 g/100 ml |
| Kalium | 150 mg/100ml |
| Vitamin A | 90 µg/100 ml |

Gemäß den Schritten 9 und 10 frägt die Steuerungseinrichtung zyklisch die Förderrate, den Medikamentennamen und die Kohlenhydratkonzentration von der Ernährungspumpe ab. Daraufhin wird in einem Schritt 11 innerhalb der Steuerungseinrichtung die Kohlenhydratrate in g/h aus der Förderrate und der Konzentration berechnet und anschließend an die Berechnungseinrichtung gemäß Schritt 12 übertragen.

In der Berechnungseinrichtung (Space Com) findet in einem Schritt 13 die Berechnung einer neuen Insulinrate und die Darstellung dieser Insulinrate auf einem Bildschirm einer Anzeigeeinrichtung für das Bedienungspersonal statt.

Bei einer Änderung der relevanten unter den Schritten 9 und 10 erfragten Eingangsparameter wird demzufolge automatisiert der Berechnungsvorgang gestartet und somit ein Vorschlag für eine neue Insulinrate errechnet. Hierbei sind die wichtigsten relevanten Eingangsparameter der Blutglukosewert, eine Änderung in der Ernährung und/oder eine Änderung des Patientengewichtes.

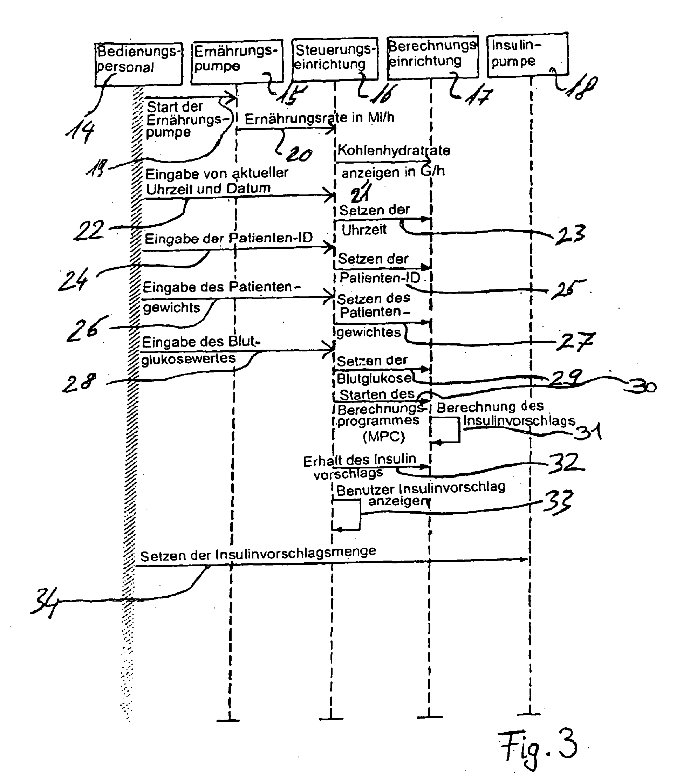

In Fig. 3 ist in einem Ablaufdiagramm des Abschnitts der Initialisierungsphase des erfindungsgemäßen Verfahrens nochmals dargestellt. Das in Fig. 3 gezeigte Ablaufdiagramm zeigt das Bedienungspersonal 14, welches bestimmte Tätigkeiten gegenüber einer Ernährungspumpe 15, einer Steuerungseinrichtung 16 und einer Insulinpumpe 18 durchführt. Eine Berechnungseinrichtung 17 steht in stetigem Datenaustausch mit der Steuerungseinrichtung 16.

In einem Schritt 19 wird von dem Bedienungspersonal 14 die Ernährungspumpe 15 gestartet. Ein weiterer Schritt 20 liefert die Förderrate bzw. Nahrungsrate von der Ernährungspumse 15 zu der Steuerungseinrichtung 16.

Die Steuerungseinrichtung 16 sendet daraufhin eine von ihr berechnete Kohlenhydratrate in einem Schritt 21 an die Berechnungseinrichtung 17.

In einem Schritt 22 findet die Eingabe der aktuellen Uhrzeit und des aktuellen Datums durch das Bedienungspersonal in die Steuerungseinrichtung 16 statt. Diese wiederum gibt diese Daten in einem Schritt 23 an die Berechnungseinrichtung weiter. Ebenso findet eine Eingabe 24 der Patienten- ID, in einem Schritt 26 des Patientengewichtes und in einem Schritt 28 de Blutglukosewertes durch das Bedienungspersonal 14 an die Steuerungseinrichtung 16 statt.

Die Steuerungseinrichtung 16 überträgt wiederum in den Schritten 25, 27 und 29 die Patienten-ID, das Patientengewicht und den Blutglukosewert an die Berechnungseinrichtung 17.

Anschließend findet eine Berechnung eines vorgeschlagenen Insulinwertes in einem Schritt 31 innerhalb der Berechnungseinrichtung 17 statt. Daraufhin fordert die Steuerungseinrichtung 16 den neu berechneten und vorgeschlagenen Insulinwert von in einem Schritt 32 von der Berechnungseinrichtung und stellt diese berechnete Insulinrate auf eine in der Steuereinrichtung 16 dargestellte Anzeigeeinrichtung in einem Schritt 33 dar. Sofern das Bedienungspersonal der vorgeschlagenen Insulinrate aus medizinischer Sicht zustimmt, findet nun die Eingabe der vorgeschlagenen Insulinrate in der Insulinpumpe 80 durch das Bedienungspersonal 14 in einem Schritt 34 statt.

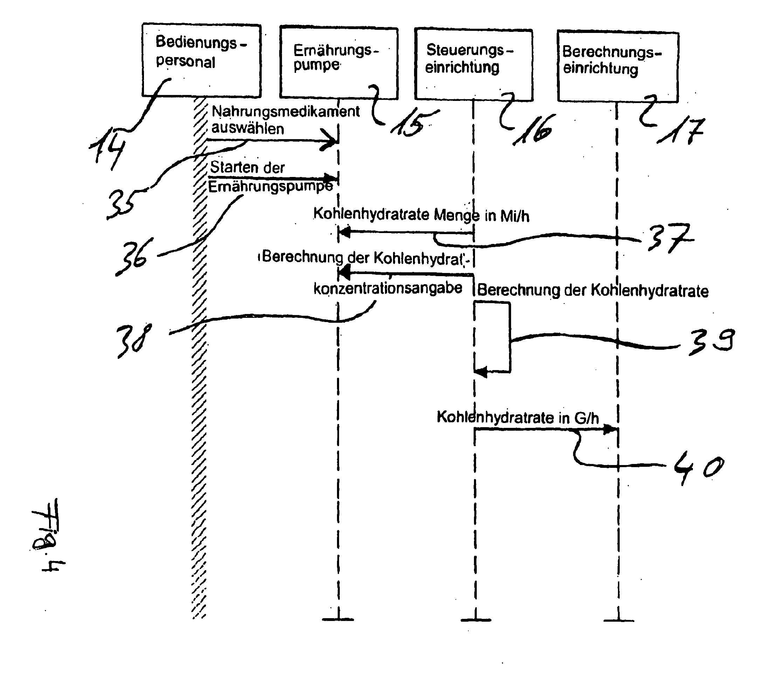

In Fig. 4 wird ein Ausschnitt des erfindungsgemäßen Verfahrens in einem Ablaufdiagramm gezeigt. In dieser Darstellung ist die automatische Einbindung der Ernährung und somit der Berechnung der Nahrungswerte in einem automatisierten Verfahrensablauf dargestellt.

Ein Bedienungspersonal 14 wählt in einem Schritt 35 eines der Nahrungsmedikamente, welches in der Ernährungspumpe 15 vorhanden ist. Anschließend findet ein Start 36 der Ernährungspumpe 15 statt.

In einem Schritt 37 wird ein Abfragesignal von der Steuerungseinrichtung 16 an die Ernährungspumpe 15 gesendet, um die Förderrate bzw. Nahrungsrate von der Ernährungspumpe 15 als Information zu erhalten. Ebenso wird in einem Schritt 38 die Kohlenhydratkonzentrationsangabe in der ausgewählten Nahrungsart der künstlichen Nahrung von der Steuerungseinrichtung 16 angefordert.

Die Steuerungseinrichtung 16 berechnet nun in einem Schritt 39 aus den vorangegangenen erhaltenen Daten gemäß den Schritten 37 und 38 eine Kohlenhydratrate und zeigt diese Kohlenhydratrate in einem Schritt 40 auf der Steuerungseinrichtung und ihrer Anzeigeeinrichtung an.

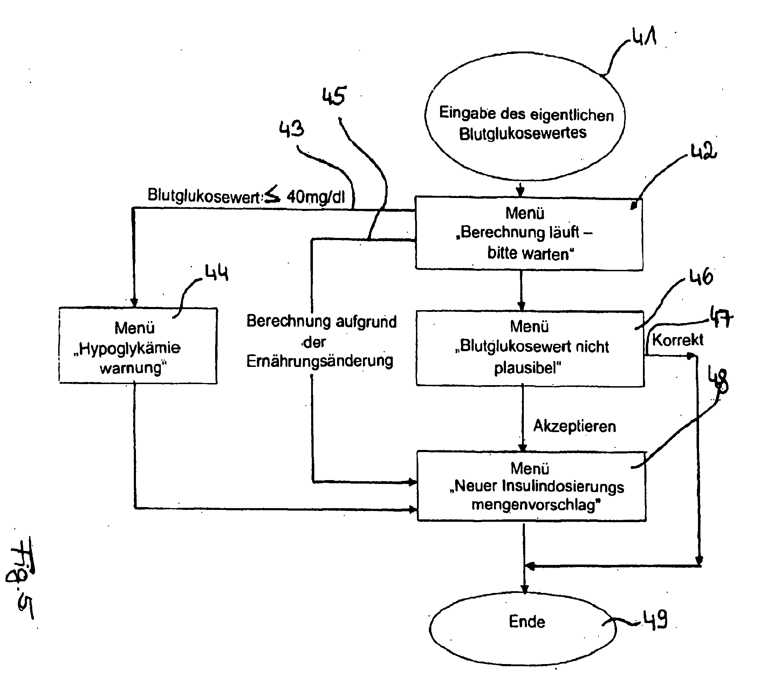

Fig. 5 zeigt in einem weiteren Flußdiagramm schematisiert einen Berechnungsablauf zur Berechnung der Insulinrate. In einem Schritt 41 wird der eigentliche gemessene Blutglukosewert- auch bei dessen Änderung- über die Eingabeeinrichtung in das System eingegeben.

Anschließend findet ein Starten der Berechnungseinrichtung durch die Steuerungseinrichtung in einem Schritt 42 statt. Während des Startvorganges wird die Anzeige "Berechnung läuft- bitte warten" angezeigt.

Wenn ein derartiger Berechnungsvorgang aufgrund einer Ernährungsänderung oder einer Gewichtsänderung des Patienten gestartet wurde, dann wird dem Bedienungspersonal sofort ein Vorschlag für eine neue Insulinrate gemäß dem Schritt 45 und dem Schritt 48 angezeigt. Den angezeigten Insulinratenwert kann das Bedienungspersonal entweder an der Insulinpumpe einstellen oder ihn ablehnen, indem eine andere Insulinrate an der Insulinpumpe eingestellt wird.

Sofern der Berechnungsmodus aufgrund eines neuen gemessenen Blutglukosewertes gestartet wurde, wird dem Bedienungspersonal eine Hypoglykämiewarnung in den Schritten 43 und 44 angezeigt, wenn der neu eingegebene Blutglukosewert weniger als 40 mg/dl ist.

Sofern der eingegebene Blutglukosewert einen derartigen Wert, der auch eine andere Größe annehmen kann, nicht unterschreitet, findet in einem Schritt 46 eine Art Plausibilitätsüberprüfung des eingegebenen Wertes hinsichtlich der weiteren der Berechnungseinrichtung vorliegenden Werte und in Abhängigkeit von einem zugrunde liegenden Patientenmodell statt.

Wenn die Plausibilitätsprüfung ergibt, dass der eingegebene Wert nicht sinnvoll in Abhängigkeit von den weiteren Werten erscheint, wird dieser Schritt 46 auf der Anzeige angezeigt. In Abhängigkeit von dem angezeigten Wert muss das Bedienungspersonal nun entscheiden, ob ein Messfehler vorliegen kann und somit der Wert zu verwerfen ist, um eine neue Messung durchzuführen, oder ob dieser Blutzuckerwert korrekt ist und somit mittels des Schrittes 47 zugelassen wird.

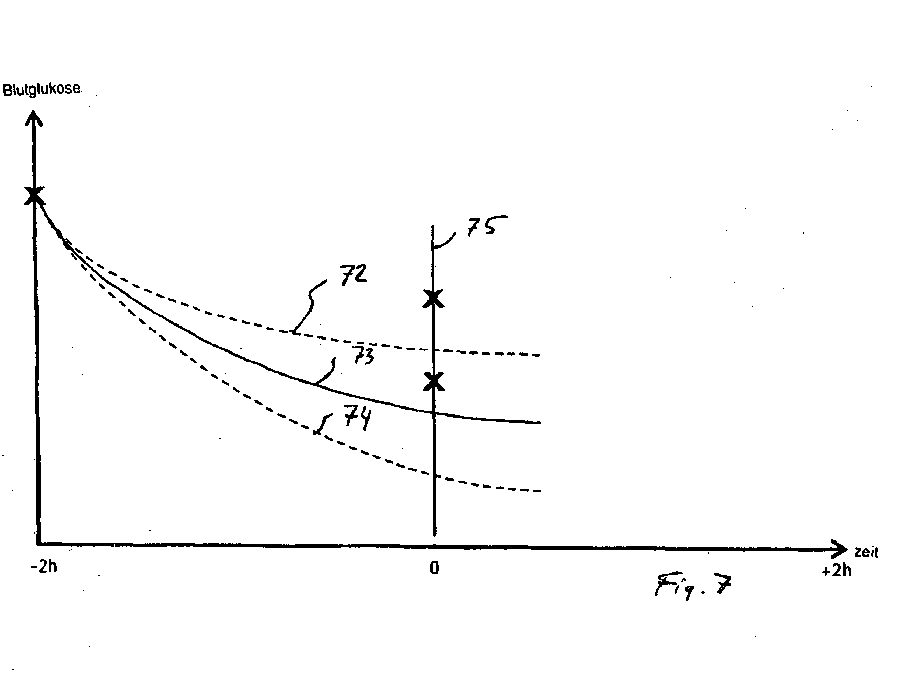

Eine Plausibilitätsprüfung erfolgt nach einem Verfahren, wie es anhand des in Fig. 7 gezeigten Diagramms näher dargestellt werden soll. In Fig. 7 wird in einem Diagramm der erwartete Verlauf des Blutglukosewertes über die nächsten vier Stunden wiedergegeben. Zum Zeitpunkt wird eine Trajektorie mit dem zu erwartenden Blutglukosewert berechnet. Dies entspricht der Kurve 73. Ebenso werden erwartete kegelartige Fehlerbereiche durch eine obere und eine untere Grenze gemäß den Kurven 72 und 74 für den Blutglukosewert berechnet und festgelegt. Sofern die gemessenen Werte, welche auf einer Linie 75 liegen, nach Ablauf von zwei Stunden innerhalb dieses Bereichskegels zwischen den Linien 72 und 74 liegen, liegt ein plausibel erscheinender Messwert vor. Außerhalb des Bereichskegels zwischen den Linien 72 und 74 liegende Messwerte werden dem Bedienungspersonal als Warnung angezeigt.

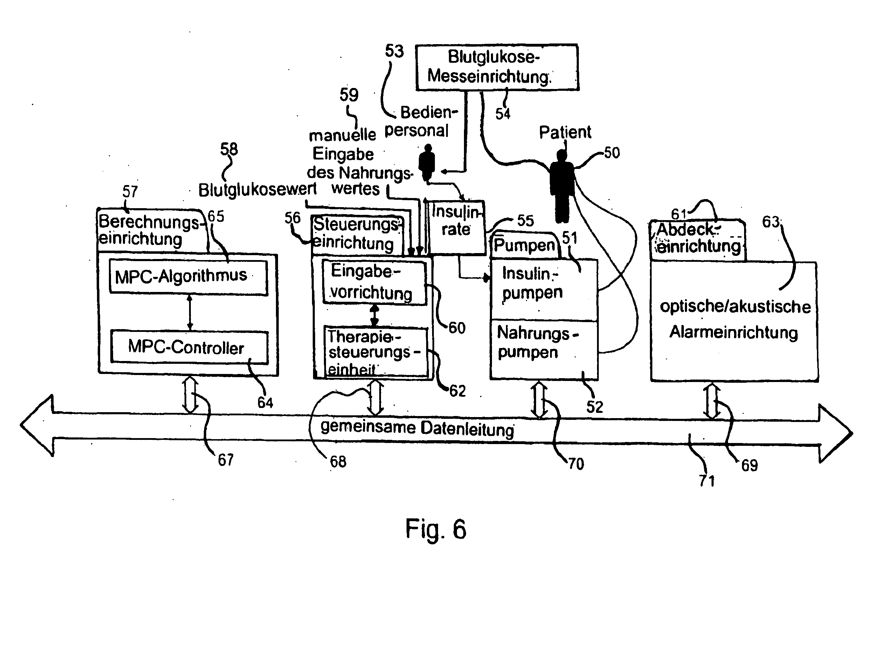

In Fig. 6 ist in einer schematischen Darstellung das erfindungsgemäße System gemäß einer Ausführungsform der Erfindung gezeigt. Das Bedienungspersonal 53 erhält mittels einer Blutglukose- Messeinrichtung 54 gemessene Blutglukosewerte. Ebenso kann das Bedienungspersonal 53 Insulinraten 55 an eine Insulinpumpe 51 übertragen bzw. dort eingeben und ggf. ebenso Nahrungswerte in der Nahrungspumpe 52 eingeben. Beide Pumpen 51 und 52 sind mit einem Patienten 50 verbunden.

Eine Steuerungseinrichtung 56 steht mit einer Berechnungseinrichtung 57 in direktem Kontakt mittels einer gemeinsamen Datenleitung 71, die hier schematisch dargestellt ist.

Ein Blutglukosewert 58 und eine manuelle Eingabe von Nahrungswerten 59 findet über eine Eingabevorrichtung 60, die auch zugleich Ausgabeeinrichtung ist, statt. Die Eingabeeinrichtung 60 steht in direktem Kontakt mit einer Therapiesteuerungseinheit 62, welche für den Austausch von Daten und deren Steuerung sowie Regelung zuständig ist. Demzufolge werden über mehrere Kanäle 68, 69, 70 und 67 sichere und unsichere Kommunikationsdaten zwischen der Steuerungseinrichtung 56 und der Berechnungseinrichtung 57 sowie den Pumpen 51, 52 und einer Alarmeinrichtung 63 ausgetauscht.

In der optischen und/oder akustischen Alarmeinrichtung 63 wird ein Alarmsignal erzeugt, sofern eine erneut anstehende Messung des Blutglukosewertes nach Ablauf eines Messzeitabstandes oder ein Aussetzen der zugeführten Insulinrate und/oder ein Aussetzen der gegebenenfalls zugeführten Nahrungswerte und/oder eine starke Veränderung des Blutglukosewertes stattfindet. Dies kann sowohl optisch als auch akustisch alarmiert werden, um Personal, welches sich momentan nicht vor Ort befindet, herbeizurufen.

In der Berechnungseinrichtung 57 wird mittels eines darin gespeicherten Berechnungsprogrammes 65 und einem damit zusammenwirkenden MPC-Controller 64 eine Berechnung der Insulinrate und ggf. weiterer Daten wie beispielsweise neuer Nahrungswerte durchgeführt.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

| | |
|---|---|
| 1 | Automatische Abfrage der Ernährungsdaten |
| 2 | Eingabe der aktuellen Uhrzeit und des Datums |
| 3 | Eingabe der Patienten- ID |
| 4 | Eingabe des Patientengewichts |
| 5 | Eingabe der Blutglukosewerte |
| 6 | Berechnen eines neuen Insulinratenvorschlags |
| 7 | Start der Insulinpumpe |
| 8 | Ernährung wird geändert |
| 9 | Steuerungseinrichtung sendet Anfragesignal |
| 10 | Steuerungseinrichtung sendet Abfragesignal |
| 11 | Steuerungseinrichtung berechnet Kohlenhydratrate |
| 12 | Steuerungseinrichtung überträgt Kohlenhydratrate an Berechnungseinrichtung |
| 13 | Berechnen der neuen Insulinrate |
| 14 | Bedienungspersonal |
| 15 | Ernährungspumpe |
| 16 | Steuerungseinrichtung |
| 17 | Berechnungseinrichtung |
| 18 | Insulinpumpe |
| 19 | Start der Ernährungspumpe |
| 20 | Überlieferung der Förderrate |
| 21 | Überlieferung der Kohlenhydratrate |
| 22 | Eingabe der Uhrzeit und des Datums |
| 23 | Setzen der Uhrzeit |
| 24 | Eingabe der Patienten- ID |
| 25 | Setzen der Patienten- ID |
| 26 | Eingabe des Patientengewichtes |
| 27 | Setzen des Patientengewichtes |
| 28 | Eingabe der Blutglukosewerte |
| 29 | Setzen der Blutglukosewerte |
| 30 | Starten des Berechnungsprogrammes |
| 31 | Berechnen der Insulinrate |
| 32 | Übersenden der Insulinrate |
| 33 | Anzeige der vorgeschlagenen Insulinrate |
| 34 | Einstellung der Insulinrate an der Insulinpumpe |
| 35 | Auswählen des Nahrungsmedikaments |
| 36 | Starten der Nahrungspumpe |
| 37 | Übertragen der Förderrate |
| 38 | Übertragen der Kohlenhydratkonzentration |
| 39 | Berechnen der Kohlenhydratrate |
| 40 | Übertragen der Kohlenhydratrate |
| 41 | Eingabe des Blutglukosewertes |
| 42 | Berechnung gestartet |
| 43 | Blutglukosewert unterschreitet bestimmten Wert |
| 44 | Hypoglykämiewarnung |
| 45 | Berechnung aufgrund Nahrungswertänderungen |
| 46 | Blutglukosewert ist nicht plausibel |
| 47 | Blutglukosewert ist korrekt |
| 48 | Neue Insulinrate wird berechnet und vorgeschlagen |
| 49 | Ende |
| 50 | Patient |
| 51 | Insulinpumpe |
| 52 | Nahrungszuführeinrichtung |
| 53 | Bedienungspersonal |
| 54 | Blutglukosemesseinrichtung |
| 55 | Insulininfusionsratenvorrichtung |
| 56 | Steuerungseinrichtung |
| 57 | Berechnungseinrichtung |
| 58 | Blutglukose |
| 59 | Daten zu der künstlichen Nahrung |
| 60 | User Interface |
| 62 | Therapiesteuerungseinheit |
| 63 | Alarmeinrichtung |
| 64 | MPC-Controller |
| 65 | Berechnungsprogramm |
| 67, 68, 69, 70 | Datenkommunikationskanäle |
| 71 | Datenkommunikationshauptkanal |
| 72, 73, 74 | Blutglukosewertverläufe |
| 75 | Gerade mit darauf angeordneten Messwerten |

## Patentansprüche

1. System zur Überwachung und Regelung von Blutglukosewerten (58) in einem Blutkreislauf mit
einer Eingangseinrichtung (14) zum Empfangen von mindestens einem gemessenen im Blutkreislauf bestehenden Blutglukosewert (58), von mindestens einem mit mindestens einer Insulinzuführeinrichtung (18, 51) dem Blutkreislauf bisher zugeführten Insulinwert, und von mindestens einem Nahrungswert (59) aus einer Mehrzahl an Daten von einer mit mindestens einer Nahrungszuführeinrichtung (15, 52) dem Blutkreislauf bisher direkt oder indirekt zugeführten Nahrung, wobei die Eingangseinrichtung (14) eine Eingabevorrichtung (60) zum Eingeben der gemessenen oder berechneten Blutglukosewerte (58) und/oder Nahrungswerte (59) und weiterer personenspezifischer Werte, wie Körpergewicht, Personenalter und dergleichen Daten aufweist;
einer Berechnungseinrichtung (17, 57) zum Berechnen (6, 13) eines neuen Insulinwertes (55) und gegebenenfalls eines neuen Nahrungswertes in Abhängigkeit des Einflusses von Insulin und gegebenenfalls Nahrung auf Blutglukosewerte und in Abhängigkeit von dem zuvor gemessenen Blutglukosewert (58), und
einer Ausgabeeinrichtung (60) zum Ausgeben der neuen Insulinwerte (55) und gegebenenfalls Nahrungswerte,
**dadurch gekennzeichnet, dass**
die von der Nahrungszuführeinrichtung (15, 52) empfangbaren Daten eine Förderrate (8) der Nahrungszuführung einschließen und die Berechnungseinrichtung (17,57) dazu eingerichtet ist, die neuen Nahrungswerte als Folge einer Unterbrechung oder Änderung einer Förderrate (8) einer Nahrungszuführung der Nahrungszuführeinrichtung (15) zu berechnen.

2. System nach Anspruch 1, worin
die Eingangs- und die Ausgabeeinrichtung in einer Steuereinrichtung (16, 56) mit Sende- und Empfangseinrichtung zusammengefasst sind.

3. System nach Anspruch 1 oder 2,
worin
die Ausgabeeinrichtung (60) an eine Anzeigeeinrichtung angeschlossen ist.

4. System nach Anspruch 1 oder 2,
worin
die Ausgabeeinrichtung (60) mit der Nahrungszuführeinrichtung (52) verbunden ist.

5. System nach Anspruch 1 oder 2,
worin
die Ausgabeeinrichtung (60) mit der Insulinzuführeinrichtung (51) verbunden ist.

6. System nach einem der Ansprüche 2-5,
worin, die Steuereinrichtung (16) eingerichtet ist, mindestens ein Abfragesignal (37, 38) zur Abfrage der Nahrungswerte an die Nahrungszuführeinrichtung (15) zu mindestens einem vorbestimmbaren Zeitpunkt zu senden.

7. System nach Anspruch 6,
worin
die abgefragten Nahrungswerte die aktuelle Förderrate der als Pumpe ausgestalteten Nahrungszuführeinrichtung (15, 52), die Nahrungsart und deren Kohlenhydratkonzentration umfassen.

8. System nach einem der vorangegangenen Ansprüche,
umfassend
eine Alarmierungseinrichtung zum optischen oder akustischen Alarmieren eines Bedieners (53) für die zeitabhängig fällige Messung (54) des Blutglukosewertes (58) im Blutkreislauf.

9. Verfahren zur Überwachung und Regelung von Blutglukosewerten in einem Blutkreislauf,
mit den Schritten:
- Starten (19, 36) mindestens einer Nahrungszuführeinrichtung (15, 52) zum direkten oder indirekten Zuführen von Nahrung in den Blutkreislauf,
- Ermitteln (1, 20, 37, 38) mindestens eines Nahrungswertes der Nahrung aus einer Mehrzahl an von der Nahrungszuführeinrichtung (15, 52) an eine Steuerungseinrichtung (16, 56) übermittelten Daten, welche eine Förderrate (8) der Nahrungszuführung einschließen,
- Übermittlung (5, 28) von gemessenen in dem Blutkreislauf bestehenden Blutglukosewerten (58) in die Steuerungseinrichtung (16, 56),
- Berechnen (6,13,31) eines Insulinwertes (55) und gegebenenfalls eines Nahrungswertes in Abhängigkeit des Einflusses von Insulin und gegebenenfalls Nahrung auf Blutglukosewerte und in Abhängigkeit von dem zuvor gemessenen Blutglukosewert (58) mittels einer Berechnungseinrichtung (17, 57), **dadurch gekennzeichnet, dass** die neuen Nahrungswerte dann berechnet werden, wenn die Nahrungszuführeinrichtung (15) die Nahrungszuführung unterbricht oder ihre Förderrate (8) ändert, und
- Ausgeben (6, 33) der berechneten neuen Insulinwerte (55) und gegebenenfalls der berechneten neuen Nahrungswerte mittels einer Ausgabeeinrichtung (60).

10. Verfahren nach Anspruch 9,
worin
die ausgegebenen berechneten Insulinwerte (55) an eine Insulinzuführeinrichtung (18, 51) übermittelt (34) oder darin eingegebenen werden.

11. Verfahren nach Anspruch 9 oder 10,
worin
die ausgegebenen berechneten Nahrungswerte an die Nahrungszuführeinrichtung (52) übertragen oder darin eingegeben werden.

12. Verfahren nach einem der Ansprüche 9-11,
worin
der Schritt des Ermittelns (1, 20, 37, 38) des Nahrungswertes umfasst:
- Abfragen (9, 37) der Förderrate, der Nahrungsart (10) und einer Kohlenhydratkonzentration (19, 38), von der Nahrungszuführeinrichtung (15) durch die Steuerungseinrichtung (16),
- Berechnen (11, 39) der Kohlenhydratrate als Nahrungswerte aus der Förderrate, der Nahrungsart und der Kohlenhydratkonzentration mittels der Steuerungseinrichtung (16),
- Übermittlung (12, 14) der Kohlenhydratrate an die Berechnungseinrichtung (17) zum Berechnen (13, 31) der neuen Insulinwerte und gegebenenfalls neuer Nahrungswerte.

13. Verfahren nach einem der Ansprüche 9 - 12,
worin
personenspezifische Daten, wie Körpergewicht, Personenalter und dergleichen zum Berechnen (13,31) der neuen Insulinwerte in die Steuerungseinrichtung (16) eingegeben werden (2, 3, 4; 22, 24, 26).

14. Verfahren nach einem der Ansprüche 9-13,
worin
eine Alarmierung eines Bedieners (53) mittels einer in der Steuereinrichtung (56) angeordneten Alarmierungseinrichtung (63) für die zeitabhängig fällige Messung (54) des Blutglukosewertes im Blutkreislauf durchgeführt wird.

15. Verfahren nach Anspruch 14,
worin
eine Alarmierung des Bedieners (53) mittels der Alarmierungseinrichtung (63) bei einer nicht vorherbestimmten Veränderung der zugeführten Nahrungswerte erfolgt.

16. Verfahren nach einem Ansprüche 9-15
worin
die von der Berechnungseinrichtung ausgegebenen Werte einem Bediener (53) angezeigt und von diesem als zutreffend bestätigt werden.

17. Verfahren nach einem der Ansprüche 9-16
worin
eine Plausibilitätsprüfung zu der Plausibilität des gemessenen Blutglukosewertes durchgeführt wird.

## Claims

1. A system for monitoring and regulating blood glucose levels (58) in a bloodstream, comprising:
an input unit (14) for receiving at least one measured blood glucose level (58) present in the bloodstream, of at least one insulin value so far supplied to the bloodstream by means of at least one insulin supply unit (18, 51), and of at least one nutritional value (59) out of a plurality of data of nutrition so far directly or indirectly supplied to the bloodstream by means of at least one nutrition supply unit (15, 52), wherein the input unit (14) contains an input device (60) for the input of the measured or calculated blood glucose levels (58) and/or nutritional values (59) and further person-specific values such as body weight and the age of a person and such data;
a calculation unit (17, 57) for calculating (6, 13) a new insulin value (55) and, if applicable, a new nutritional value dependent on the effect of insulin and, if applicable, of the nutrition on the blood glucose levels and dependent on the previously measured blood glucose level (58), and
an output unit (60) for outputting the new insulin value (55) and, if applicable, nutritional values,
**characterized in that**
the data receivable by the nutrition supply unit (15, 52) include a delivery rate (8) of the nutrition supply and the calculation unit (17, 57) is set to calculate the new nutritional values as a result of an interruption or change of a delivery rate (8) of a nutrition supply of the nutrition supply unit (15).

2. A system according to claim 1, wherein
the input and output units are combined within a control unit (16, 56) with a transmission and reception unit.

3. A system according to claim 1 or 2, wherein
the output unit (60) is attached to a display unit.

4. A system according to claim 1 or 2, wherein
the output unit (60) is connected to the nutrition supply unit (52).

5. A system according to claim 1 or 2, wherein
the output unit (60) is connected to the insulin supply unit (51).

6. A system according to one of the claims 2-5, wherein
the control unit (16) is set to transmit at least one polling signal (37, 38) for polling the nutritional values to the nutrition supply unit (15) at least at one predeterminable point in time.

7. A system according to claim 6, wherein
the polled nutritional values comprise the current delivery rate of the nutrition supply unit (15, 52) embodied as a pump, the type of nutrition and the carbohydrate concentration thereof.

8. A system according to one of the preceding claims, comprising an alarm unit for visually or acoustically alarming an operator (53) that it is time for the due measurement (54) of the blood glucose level (58) in the bloodstream.

9. A method for monitoring and regulating blood glucose levels in a bloodstream,
comprising the following steps:
- starting (19, 36) at least one nutrition supply unit (15, 52) for directly or indirectly supplying nutrition into the bloodstream,
- determining (1, 20, 37, 38) at least one nutritional value of the nutrition from a plurality of data transferred by the nutrition supply unit (15, 52) to a control unit (16, 56), which include a delivery rate (8) of the nutrition supply,
- transferring (5, 28) measured blood glucose levels (58) present in the bloodstream to the control unit (16, 56),
- calculating (6, 13, 31) an insulin value (55) and, if applicable, a nutritional value dependent on the effect of insulin and, if applicable, nutrition on blood glucose levels and dependent on the previously measured blood glucose level (58) by means of a calculating unit (17, 57), **characterized in that** the new nutritional values are calculated when the nutrition supply unit (15) interrupts the nutrition supply or changes its delivery rate (8), and
- outputting (6, 33) the calculated new insulin values (55) and, if applicable, the calculated new nutritional values by means of an output unit (60).

10. A method according to claim 9, wherein
the output calculated insulin values (55) are transferred (34) to an insulin supply unit (18, 51) or input therein.

11. A method according to claim 9 or 10, wherein
the output calculated nutritional values are transferred to the nutrition supply unit (52) or input therein.

12. A method according to one of the claims 9-11, wherein
the step of determining (1, 20, 37, 38) the nutritional value comprises the following:
- polling (9, 37) the delivery rate, the type of nutrition (10) and a carbohydrate concentration (19, 38) from the nutrition supply unit (15) by the control unit (16),
- calculating (11, 39) the carbohydrate rate as nutritional values from the delivery rate, the type of nutrition and the carbohydrate concentration by means of the control unit (16),
- transferring (12, 14) the carbohydrate rate to the calculation unit (17) for calculating (13, 31) new insulin values and, if applicable, new nutritional values.

13. A method according to one of the claims 9-12, wherein, person-specific data such as body weight, age of the person and the like are input (2, 3, 4; 22, 24, 26) into the control unit (16) for calculating (13, 31) the new insulin values.

14. A method according to one of the claims 9-13, wherein,
alarming an operator (53) is carried out by means of an alarm unit (63) disposed in the control unit (56) to indicate that it is time for the due measurement (54) of the blood glucose level in the bloodstream.

15. A method according to claim 14, wherein,
alarming an operator (53) is executed by means of the alarm unit (63) in the event of a not-predetermined change of the supplied nutritional values.

16. A method according to one of the claims 9 -15, wherein,
the values output by the calculation unit are displayed to an operator (53) and confirmed by the operator as applicable[/correct].

17. A method according to one of the claims 9-16, wherein,
a plausibility check with regard to the plausibility of the measured blood glucose level is carried out.

## Revendications

1. Système de surveillance et de régulation de taux de glycémie (58) dans une circulation sanguine avec
- un dispositif d'entrée (14) destiné à recevoir au moins un taux de glycémie (58) existant dans la circulation sanguine et mesuré, au moins un taux d'insuline apporté jusqu'au moment concerné dans la circulation sanguine avec au moins un dispositif d'apport d'insuline (18, 51) et au moins un taux d'alimentation (59) issu d'une pluralité de données d'une alimentation apportée directement ou indirectement jusqu'au moment concerné dans la circulation sanguine avec au moins un dispositif d'apport d'alimentation (15, 52), dans lequel le dispositif d'entrée (14) présente un module d'introduction (60) destiné à l'introduction des taux de glycémie (58) et/ou des taux d'alimentation (59) et de paramètres supplémentaires spécifiques à la personne, mesurés ou calculés, comme le poids corporel, l'âge de la personne et des données similaires ;
- un dispositif de calcul (17, 57) destiné à calculer (6, 13) un nouveau taux d'insuline (55), et le cas échéant, un nouveau taux d'alimentation en fonction de l'influence de l'insuline, et le cas échéant, de l'alimentation sur les taux de glycémie et en fonction du taux de glycémie (58) mesuré antérieurement ; et
- un dispositif de sortie (60) destiné à produire les nouveaux taux d'insuline (55), et le cas échéant les nouveaux taux d'alimentation,
**caractérisé en ce que**
les données que le dispositif d'apport d'alimentation (15, 52) peut recevoir incluent un débit (8) de l'apport d'alimentation, et le dispositif de calcul (17, 57) est réalisé de façon à calculer les nouveaux taux d'alimentation à la suite d'une interruption ou d'une modification du débit (8) d'un apport d'alimentation du dispositif d'apport d'alimentation (15).

2. Système selon la revendication 1,
dans lequel les dispositifs d'entrée et de sortie sont réunis dans un dispositif de commande (16, 56) avec un dispositif d'émission et de réception.

3. Système selon la revendication 1 ou 2,
dans lequel le dispositif de sortie (60) est raccordé à un dispositif d'affichage.

4. Système selon la revendication 1 ou 2,
dans lequel le dispositif de sortie (60) est relié au dispositif d'apport d'alimentation (52).

5. Système selon la revendication 1 ou 2,
dans lequel le dispositif de sortie (60) est relié au dispositif d'apport d'insuline (51).

6. Système selon l'une des revendications 2 à 5,
dans lequel le dispositif de commande (16) est conçu de façon à envoyer au moins un signal de demande (37, 38) afin de demander les taux d'alimentation au dispositif d'apport d'alimentation (15) à au moins un moment pouvant être prédéterminé.

7. Système selon la revendication 6,
dans lequel les taux d'alimentation demandés comprennent le débit actuel du dispositif d'apport d'alimentation (15, 52) réalisé sous forme de pompe, le type d'alimentation et sa teneur en hydrates de carbone.

8. Système selon l'une des revendications précédentes,
comprenant un dispositif d'alarme destiné à adresser à un opérateur (53) une alarme visuelle ou acoustique pour la mesure planifiée en fonction du temps (54) du taux de glycémie (58) dans la circulation sanguine.

9. Procédé de surveillance et de régulation de taux de glycémie dans une circulation sanguine,
comprenant les étapes suivantes :
- démarrage (19, 36) d'au moins un dispositif d'apport d'alimentation (15, 52) destiné à l'apport direct ou indirect d'alimentation dans la circulation sanguine ;
- détermination (1, 20, 37, 38) d'au moins un taux d'alimentation de l'alimentation à partir d'une pluralité de données transmises par le dispositif d'apport d'alimentation (15, 52) à un dispositif de commande (16, 56), lesquelles données incluent un débit (8) de l'apport d'alimentation ;
- transmission (5, 28) de taux de glycémie (58) existants dans la circulation sanguine et mesurés, au dispositif de commande (16, 56) ;
- calcul (6, 13, 31) d'un taux d'insuline (55), et le cas échéant d'un taux d'alimentation en fonction de l'influence de l'insuline, et le cas échéant de l'alimentation, sur les taux de glycémie, et en fonction du taux de glycémie (58) mesuré antérieurement au moyen d'un dispositif de calcul (17, 57), **caractérisé en ce que** les nouveaux taux d'alimentation sont calculés lorsque le dispositif d'apport d'alimentation (15) interrompt l'apport d'alimentation ou modifie son débit (8), et
- production (6, 33) des nouveaux taux d'insuline (55) calculés, et le cas échéant des nouveaux taux d'alimentation calculés, au moyen d'un dispositif de sortie (60).

10. Procédé selon la revendication 9,
dans lequel les taux d'insuline (55) calculés produits sont transmis (34) à un dispositif d'apport d'insuline (18, 51) ou introduits dans celui-ci.

11. Procédé selon la revendication 9 ou 10,
dans lequel les taux d'alimentation calculés produits sont transférés au dispositif d'apport d'alimentation (52) ou introduits dans celui-ci.

12. Procédé selon l'une des revendications 9 à 11,
dans lequel l'étape de la détermination (1, 20, 37, 38) du taux d'alimentation comprend :
- la demande (9, 37) du débit, du type d'alimentation (10) et d'une teneur en hydrates de carbone (19, 38) du dispositif d'apport d'alimentation (15) via le dispositif de commande (16),
- le calcul (11, 39) du débit d'hydrates de carbone comme taux d'alimentation à partir du débit, du type d'alimentation et de la teneur en hydrates de carbone au moyen du dispositif de commande (16),
- la transmission (12, 14) du débit d'hydrates de carbone au dispositif de calcul (17) en vue du calcul (13, 31) des nouveaux taux d'insuline, et le cas échéant des nouveaux taux d'alimentation.

13. Procédé selon l'une des revendications 9 à 12,
dans lequel des données spécifiques à la personne, comme le poids corporel, l'âge de la personne et des données similaires, sont introduites (2, 3, 4 ; 22, 24, 26) dans le dispositif de commande (16) en vue du calcul (13, 31) des nouveaux taux d'insuline.

14. Procédé selon l'une des revendications 9 à 13,
dans lequel une alarme est adressée à un opérateur (53) au moyen d'un dispositif d'alarme (63) agencé dans le dispositif de commande (56) pour la mesure planifiée en fonction du temps (54) du taux de glycémie dans la circulation sanguine.

15. Procédé selon la revendication 14,
dans lequel une alarme est adressée à l'opérateur (53) au moyen du dispositif d'alarme (63) dans le cas d'une modification non déterminée préalablement des taux d'alimentation apportés.

16. Procédé selon l'une des revendications 9 à 15,
dans lequel les taux produits par le dispositif de calcul sont adressés à un opérateur (53) et confirmés par celui-ci comme étant pertinents.

17. Procédé selon l'une des revendications 9 à 16,
dans lequel un contrôle de plausibilité est exécuté sur la plausibilité du taux de glycémie mesuré.
